# FASCICULE DE BREVET EUROPEEN

(11) **EP 0 654 463 B1**
(45) Date de publication et mention de la délivrance du brevet: **28.04.1999**
(21) Numéro de dépôt: 94402487.6
(22) Date de dépôt: 04.11.1994
(51) Int. Cl.: C07C 201/16

(54) **Elimination de composés du type nitrocrésols d'un mélange comprenant des composés du type nitroaromatiques**
Entfernung von Nitrocresol Verbindungen aus einer nitroaromatischen Verbindung enthaltenden Mischung
Elimination of nitrocresol compounds from a mixture containing nitroaromatic compounds

(30) Priorité: 19.11.1993 FR 9313823
(43) Date de publication de la demande: 24.05.1995
(73) Titulaire: RHODIA CHIMIE, 92408 Courbevoie Cédex (FR)
(72) Inventeur: Gacon-Camoz, Antoinette, F-69800 Saint Priest (FR); Metivier, Pascal, F-69110 Sainte Foy Les Lyon (FR)
(74) Mandataire: Dubruc, Philippe

(56) Documents cités:
- FR-A- 498 947
- US-A- 4 482 769
- US-A- 4 604 214

## Description

La présente invention a trait à un procédé de purification de composés du type nitroaromatiques, obtenus par nitration de composés aromatiques par l'acide nitrique, en présence d'acide sulfurique.

Plus particulièrement, l'invention consiste à éliminer des composés de la famille des crésols, substitués par des groupements nitro, existants en mélange avec les composés nitroaromatiques précités.

D'une façon classique, l'obtention de composés nitroaromatiques, et plus particulièrement de composés aromatiques comprenant deux groupements nitro, est effectuée en deux étapes. Ainsi, dans une première étape les réactifs aromatiques sont mis en présence d'un mélange d'acides nitrique et sulfurique. Le mélange réactionnel est alors décanté et la phase organique, comprenant les composés aromatiques mononitrés, est mise en contact, dans une seconde étape, avec un mélange acide nitrique/acide sulfurique pour donner les composés aromatiques correspondants, substitués par deux groupements nitro.

Cependant, le mélange issu de cette deuxième opération comprend des sous produits comme notamment des composés du type des crésols, substitués plus particulièrement par trois groupements nitro (appelés par la suite nitrocrésols).

Or ces sous produits doivent être séparés des composés nitroaromatiques avant que ces derniers ne soient engagés dans une réaction ultérieure. En effet, et pour prendre l'exemple du procédé de préparation d'amines aromatiques à partir des composés nitroaromatiques précités, les nitrocrésols sont reconnus comme poison des catalyseurs mis en oeuvre pour la réaction de réduction des groupements nitro en groupements amine.

Dans le but séparer les nitrocrésols des composés nitroaromatiques, il est connu de traiter ledit mélange avec une base et de l'eau, simultanément ou successivement, afin de transformer les nitrocrésols en sels, puis de séparer la phase organique, comprenant essentiellement les composés nitroaromatiques, de la phase aqueuse, à base des sels des nitrocrésols.

Mais l'inconvénient à opérer comme indiqué ci-dessus réside dans le fait que les effluents aqueux comprennent des quantités jugées trop importantes en sels de nitrocrésols, composés présentant une demande chimique en oxygène très élevée. Par conséquent, les effluents les contenant ne peuvent pas être rejetés tels quels.

De ce fait, il est nécessaire d'effectuer des opérations de traitement chimique ou biochimique de façon à diminuer la quantité de sels de nitrocrésols rejetée ou bien encore d'incinérer de tels effluents. Il est clair que ces opérations occasionnent des frais supplémentaires.

Le brevet US 4 604 214 par exemple, propose un procédé de traitement chimique d'élimination des sels de nitrocrésols présents dans des effluents aqueux. Ces effluents sont obtenus par décantation du mélange réactionnel issu de la nitration du toluène, suivie d'un traitement avec une base, de manière à transformer les nitrocrésols en sels hydrosolubles. La phase aqueuse, comprenant lesdits sels hydrosolubles de nitrocrésols, est ensuite séparée, par décantation, de la phase organique comprenant les dinitrotoluènes. La phase aqueuse est alors acidifiée puis traitée avec le réactif de Fenton (ions ferreux, peroxyde d'hydrogène) afin de détruire les composés organiques présents. Cependant, ce traitement dégrade aussi les dinitrotoluènes qui on été entraînés dans la phase aqueuse lors de la décantation après le traitement basique.

La présente invention a pour but de pallier les inconvénients précités.

Elle propose, tout d'abord, une méthode permettant de diminuer la quantité globale de nitrocrésols résiduels présente dans les effluents aqueux rejetés, dans une mesure comparable à celle obtenue selon les voies connues consistant à traiter les effluents.

Par ailleurs, l'invention permet d'atteindre un tel résultat en mettant en oeuvre un procédé simplifié, puisqu'une étape est évitée, et plus économique en temps et en investissements.

Ainsi, la présente invention a pour objet un procédé d'élimination de composés du type nitrocrésols contenus dans un mélange, à base de composés du type nitroaromatiques, différents des trinitrotoluènes ; ledit mélange étant obtenu par nitration de composés aromatiques par l'acide nitrique en présence d'acide sulfurique. Le procédé selon l'invention consiste à mettre en contact ledit mélange avec d'une part, un agent oxydant choisi parmi le peroxyde d'hydrogène, l'eau de Javel, ou leur mélange, et d'autre part un composé susceptible de transformer en sels hydrosolubles les nitrocrésols contenus dans le mélange.

Comme on peut le constater, le traitement d'élimination selon l'invention n'est pas effectué sur les effluents aqueux mais sur le milieu réactionnel lui-même. Par milieu réactionnel, on entend un mélange comprenant en majorité les composés nitroaromatiques, les nitrocrésols se trouvant présents au titre d'impuretés, c'est-à-dire à hauteur d'un millier de parties par million environ.

Par conséquent, un traitement ultérieur (chimique ou biochimique) des eaux est devenu inutile pour ce qui concerne les nitrocrésols. En effet, une simple décantation du milieu réactionnel traité selon l'invention, suivie d'une étape de séparation des phases aqueuse et organique résultantes, représentent des traitements suffisants pour ce qui concerne les nitrocrésols. Il est toutefois à noter que d'autres traitements classiques des eaux, avant leur rejet, ne sont pas exclus.

D'une façon totalement surprenante, on a constaté, en outre, que le rendement global de la réaction d'obtention des composés nitroaromatiques n'était pas affecté par le traitement selon l'invention. Il est rappelé que la pratique traditionnelle consiste à traiter la phase aqueuse débarrassée des composés nitroaromatiques, pour en éliminer les nitrocrésols présents. Il n'était donc pas évident, pour l'homme du métier, de mettre en oeuvre le procédé d'élimination des nitrocrésols alors que ceux-ci se trouvent toujours en mélange avec les composés nitroaromatiques fabriqués. En effet, alors que l'on aurait pu s'attendre à ce qu'un tel traitement de destruction ne soit pas sélectif et entraîne de ce fait une destruction d'une partie non négligeable des composés nitroaromatiques présents, un tel fait n'a pas été constaté. Pour être plus précis, on a noté que le traitement selon l'invention permettait d'obtenir le même rendement global en composés nitroaromatiques que les procédés de l'art antérieur. En d'autres termes il n'a pas été constaté de pertes supplémentaires en composés nitroaromatiques par mise en oeuvre du procédé selon l'invention, en comparaison avec les procédés de l'art antérieur.

Mais d'autres avantages et caractéristiques de l'invention apparaîtront plus clairement à la lecture de la description et des exemples qui vont suivre.

Ainsi qu'il a été expliqué auparavant, la présente invention a trait à un procédé de d'élimination de composés du type nitrocrésols contenus dans un mélange comprenant en outre des composés du type nitroaromatiques, différents des trinitrotoluènes ; le mélange étant obtenu par nitration de composés aromatiques avec l'acide nitrique en présence d'acide sulfurique.

Le procédé selon l'invention consiste donc à mettre en contact ledit mélange avec un agent oxydant choisi parmi le peroxyde d'hydrogène, l'eau de Javel ou leur mélange.

Selon un mode de réalisation particulier de l'invention, l'agent oxydant est le peroxyde d'hydrogène.

La quantité d'agent oxydant ajouté, exprimée en nombre de moles, est au moins égale au nombre de moles de composés du type nitrocrésols présents dans le milieu.

Selon un mode plus particulier, le procédé de l'invention est réalisé en présence d'un très large excès d'agent oxydant par rapport à la quantité de composés du type nitrocrésols présents dans le mélange à traiter.

De préférence, le nombre de moles d'agent oxydant utilisé dans le procédé est compris entre 15 et 1000 moles par mole de nitrocrésols, et encore plus particulièrement compris entre 15 et 500 moles par mole de nitrocrésols.

L'agent oxydant peut se présenter sous forme pure ou diluée dans tout solvant approprié, l'eau étant préférée.

Dans le cas d'une utilisation de l'agent oxydant sous une forme diluée, toute concentration de celui-ci peut être mise en oeuvre. Cependant, pour des raisons de cinétique ou d'économie, la concentration dudit agent dans la solution est de préférence comprise entre 5 et 50 % exprimée en poids.

Le second agent nécessaire pour la mise en oeuvre du procédé est constitué par un agent neutralisant. Par agent neutralisant, on entend tout composé susceptible de transformer en sels hydrosolubles les nitrocrésols contenus dans le mélange.

Ainsi, plus particulièrement l'agent neutralisant est choisi parmi les hydroxydes, les carbonates, les bicarbonates de métal alcalin, de métal alcalino-terreux ou leurs mélanges.

Selon un mode de réalisation préféré de l'invention, l'agent neutralisant est choisi parmi les hydroxydes de métal alcalin. Ainsi, la soude et la potasse sont des agents neutralisants particulièrement convenables pour la mise en oeuvre de l'invention.

Ledit agent peut de même se présenter sous forme pure ou diluée dans tout solvant approprié, l'eau étant préférée.

Dans le cas d'une utilisation de l'agent neutralisant sous une forme diluée, la concentration dudit agent peut varier dans une large gamme. Elle est de préférence comprise entre 5 et 50 % exprimé en poids.

La quantité d'agent neutralisant mise en jeu dans le procédé est telle que le pH de la phase aqueuse est plus particulièrement compris entre 1 et 9. Il est cependant à noter qu'opérer dans des conditions pour lesquelles le pH de la phase aqueuse se trouverait en dehors de la gamme indiquée est envisageable.

Le mélange à purifier peut comprendre en outre un co-solvant des composés nitroaromatiques fabriqués.

Plus particulièrement, on peut citer les composés aromatiques en C₆-C₁₀, éventuellement substitués par des radicaux hydrocarbonés, et/ou halogénés. A titre d'exemple, on peut mentionner le toluène, le cumène, le chlorobenzène.

Selon une première variante, le mélange directement issu de la réaction des composés aromatiques avec l'acide nitrique en présence d'acide sulfurique, subit une étape de décantation. La phase aqueuse, comprenant essentiellement le mélange des acides nitrique et sulfurique est séparée de la phase organique, à base des composés nitroaromatiques et des nitrocrésols. Ladite phase organique est alors traitée conformément au présent procédé.

Selon une seconde variante du procédé de l'invention, le mélange issu de la réaction d'obtention des composés nitroaromatiques, ayant de préférence subi le cycle décantation-séparation précité, est lavé à l'eau, de façon à éliminer les acides résiduels.

D'une façon classique, cette opération est réalisée sous agitation, à une température comprise entre 20 et 90 °C, pour une durée variant de quelques minutes à 2 heures.

Le mélange est ensuite décanté, puis la phase organique, comprenant les composés nitroaromatiques à purifier, est séparée de la phase aqueuse.

Il est à noter que cette seconde variante peut être reproduite une ou plusieurs fois.

La mise en contact du mélange à purifier et des deux agents précités a lieu de manière connue en soi.

On peut donc mettre en contact l'agent oxydant puis l'agent neutralisant, avec ledit mélange ; ou procéder à l'inverse. Il est à noter que l'introduction simultanée des deux agents est envisageable.

Dans un tel cas, les deux agents peuvent être introduits dans le milieu réactionnel sous la forme d'un mélange ou bien sous une forme de flux séparés.

Par ailleurs, on préfère introduire les agents dans le mélange à purifier, bien que le contraire ne soit pas exclu, et ce, quel que soit le mode d'introduction choisi des agents (simultané ou successif).

La mise en contact est effectuée de préférence sous agitation.

La température lors de l'opération est comprise entre 30 et 90°C et plus particulièrement entre 40 et 75°C.

Le procédé selon l'invention peut être avantageusement mis en oeuvre sous une pression comprise entre 0,5 et 10 bar et plus particulièrement à une pression voisine de la pression atmosphérique.

La durée de la mise en contact varie habituellement entre 10 minutes et 2 heures.

Le procédé de destruction des nitrocrésols selon l'invention peut avoir lieu, indifféremment, en une ou plusieurs étapes successives.

Après la mise en contact, le mélange est décanté puis la phase organique est séparée de la phase aqueuse.

La phase organique ainsi obtenue peut ensuite être lavée avec de l'eau.

D'une façon classique, cette opération est réalisée sous agitation, à une température comprise entre 20 et 90 °C, pour une durée variant de quelques minutes à 2 heures.

Le mélange résultant est ensuite décanté puis la phase organique comprenant les composés nitroaromatiques purifiés, est séparée de la phase aqueuse.

Il est noter que le procédé selon l'invention peut être mis en oeuvre de façon avantageuse en continu ou en discontinu.

Dans le second cas, et notamment pour des raisons évidentes d'économie du procédé, un recyclage de l'un et/ou l'autre des deux agents utilisés est à prévoir.

La présente invention a par ailleurs trait à l'utilisation des composés nitroaromatiques ainsi purifiés pour l'obtention de diamines aromatiques.

Généralement, cette réaction correspond à une réduction des groupements nitro en amines.

Les composés nitroaromatiques, le cas échéant après avoir subi une étape de séchage par tout moyen connu, sont mis à réagir avec de l'hydrogène, pur ou dilué, en présence d'un catalyseur classique d'hydrogénation, comme par exemple le nickel de Raney ou encore des catalyseurs à base de platine.

Habituellement la température de réaction se situe entre 100 et 200 °C et la pression durant la réaction est comprise entre 1 et 150 bar.

Des exemples concrets mais non limitatifs de l'invention vont maintenant être présentés.

### EXEMPLES

### Exemple 1 comparatif

On fait réagir du toluène avec de l'acide nitrique, en présence d'acide sulfurique. Le mélange résultant est lavé avec de l'eau de façon à éliminer les acides minéraux précités. Le mélange est décanté et la phase organique récupérée comprend des nitrotoluènes à purifier avec environ 500 ppm de nitrocrésols.

10,6 g du mélange ainsi obtenu est additionné de 10 g de toluène.
On introduit sous agitation 20 ml d'une solution d'eau puis de la soude à 36 % pour que le pH de la phase aqueuse soit d'environ 7.
Le mélange ainsi obtenu est maintenu sous agitation pendant 2 heures, à une température de 70°C.

Le mélange est décanté et l'on obtient une phase organique jaune clair et une phase aqueuse rouge vif.
La composition des deux phases et la teneur en nitrocrésols dans chacune d'elles sont analysées pu chromatographie en phase liquide.

| | taux de nitrocrésols résiduels | | |
|---|---|---|---|
| | (1) | (2) | (3) |
| phase aqueuse | 87 % | 98 % | 100 % |
| phase organique | 13% | 2% | 0 % |
| taux de destruction | 0 % | 0 % | 0 % |

| | | | |
|---|---|---|---|
| (1) 2,4,6 métatrinitrocrésol | | | |
| (2) 3,4,6 orthotrinitrocrésol | | | |
| (3) 2,3,6 paratrinitrocrésol | | | |

Le taux de destruction global des nitrocrésols est nul.

### Exemple 2

On fait réagir du toluène avec de l'acide nitrique, en présence d'acide sulfurique. Le mélange résultant est lavé avec de l'eau de façon à éliminer les acides minéraux précités. Le mélange est décanté et la phase organique récupérée comprend des nitrotoluènes à purifier avec environ 500 ppm de nitrocrésols.

10 g du mélange ainsi obtenu sont mis en solution dans 10 g de toluène.
On introduit sous agitation 20 ml d'une solution d'eau oxygénée à 10 %.
Puis on ajoute de la soude à 36 % pour que le pH de la phase aqueuse soit d'environ 7.
Le mélange ainsi obtenu est maintenu sous agitation pendant 2 heures, à une température de 70°C.

Le mélange est décanté et l'on obtient deux phases jaune clair.
La composition des deux phases et la teneur en nitrocrésols dans chacune d'elles sont analysées pu chromatographie en phase liquide.

Les résultats sont rassemblés dans le tableau ci-dessous :

| | taux de nitrocrésols résiduels | | |
|---|---|---|---|
| | (1) | (2) | (3) |
| phase aqueuse | 36 % | 0 % | 0 % |
| phase organique | 4 % | 2,5 % | 0 % |
| taux de destruction | 60 % | 97,5 % | 100 % |

Le taux de destruction global des trinitrocrésols est de 86 %.
Par ailleurs la quantité de dinitrotoluènes dans la phase organique, déterminée pu chromatographie en phase gazeuse, est identique à celle trouvée dans l'exemple 1 (rapportée à la même quantité de dinitrotoluènes initiale).

### Exemple 3

On fait réagir du toluène avec de l'acide nitrique, en présence d'acide sulfurique. Le mélange résultant est lavé avec de l'eau de façon à éliminer les acides minéraux précités. Le mélange est décanté et la phase organique récupérée comprend des nitrotoluènes à purifier avec environ 500 ppm de nitrocrésols.

10 g du mélange obtenu sont mis en solution dans 10 g de toluène.
On introduit sous agitation 20 ml d'une solution d'eau oxygénée à 10 %.
Puis on ajoute de l'acide sulfurique pour que le pH de la phase aqueuse soit d'environ 1.
Le mélange ainsi obtenu est maintenu sous agitation pendant 2 heures, à une température de 70°C.

Le mélange est décanté et l'on obtient deux phases jaune clair.
La composition des deux phases et la teneur en nitrocrésols dans chacune d'elles sont analysées par chromatographie en phase liquide.

| | taux de nitrocrésols résiduels | | |
|---|---|---|---|
| | (1) | (2) | (3) |
| phase aqueuse | 29 % | 0 % | 0 % |
| phase organique | 7 % | 1 % | 0 % |
| taux de destruction | 63 % | 99 % | 100 % |

Le taux de destruction global des nitrocrésols est de 87 %.

La quantité de dinitrotoluènes dans la phase organique, déterminée par chromatographie en phase gazeuse, est identique à celle trouvée dans l'exemple 1 (rapportée à la même quantité de dinitrotoluènes initiale).

## Revendications

1. Procédé d'élimination de composés du type nitrocrésols contenus dans un mélange à base de composés du type nitroaromatiques, différents des trinitrotoluènes, lesdits composés nitroaromatiques étant obtenus par nitration de composés aromatiques avec l'acide nitrique en présence d'acide sulfurique, caractérisé en ce que l'on met en contact ledit mélange avec d'une part, un agent oxydant choisi parmi le peroxyde d'hydrogène, l'eau de Javel, ou leur mélange, et d'autre part un composé susceptible de transformer en sels hydrosolubles les nitrocrésols contenus dans le mélange.

2. Procédé selon la revendication précédente, caractérisé en ce que l'on utilise le peroxyde d'hydrogène en tant qu'agent oxydant.

3. Procédé selon l'une quelconque des revendications précédentes, caractérisé en ce que la quantité de composé susceptible de transformer les nitrocrésols en sels hydrosolubles est telle que le pH de la phase aqueuse lors de la mise en contact est compris entre 1 et 9.

4. Procédé selon l'une quelconque des revendications précédentes, caractérisé en ce que l'on utilise, en tant que composé susceptible de transformer les nitrocrésols en sels hydrosolubles, les hydroxydes, les carbonates, les bicarbonates de métal alcalin, de métal alcalino-terreux.

5. Procédé selon la revendication précédente, caractérisé en ce que l'on utilise les hydroxydes de métal alcalin.

6. Procédé selon l'une quelconque des revendications précédentes, caractérisé en ce que l'on utilise un mélange provenant du milieu réactionnel issu de la nitration précitée, ayant préalablement été séparé de la phase aqueuse par une étape de décantation-séparation.

7. Procédé selon l'une quelconque des revendications 1 à 5, caractérisé en ce que l'on utilise un mélange réactionnel issu de la nitration précitée, de préférence séparé au préalable de la phase aqueuse par une étape de décantation-séparation, ayant été lavé à l'eau, puis séparé de la phase aqueuse par une étape de décantation-séparation.

8. Procédé selon l'une quelconque des revendications précédentes, caractérisé en ce que l'on utilise une quantité d'agent oxydant comprise entre 15 et 1000 moles, et plus particulièrement une quantité comprise entre 15 et 500 moles, par mole de composés du type nitrocrésols présents dans le mélange.

9. Procédé selon l'une quelconque des revendications précédentes, caractérisé en ce que l'on lave à l'eau la phase organique obtenue après élimination des nitrocrésols et que l'on sépare celle-ci de la phase organique, par une étape de décantation-séparation.

10. Procédé de préparation de composés aminés aromatiques, caractérisé en ce que l'on effectu le procédé selon la revendication 9 et l'on met en contact la phase organique obtenue, le cas échéant préalablement séchée, avec de l'hydrogène, pur ou dilué, en présence d'un catalyseur d'hydrogénation.

## Claims

1. A process for eliminating nitrocresol type compounds contained in a mixture of nitroaromatic compounds other than trinitrotoluenes, said nitroaromatic compounds being obtained by nitrating aromatic compounds with nitric acid in the presence of sulphuric acid, characterized in that said mixture is brought into contact with a oxidising agent selected from hydrogen peroxide or Javel water, or a mixture thereof, and with a compound which can transform the nitrocresols contained in the mixture into hydrosoluble salts.

2. A process according to the preceding claim, characterized in that hydrogen peroxide is used as the oxidising agent.

3. A process according to any one of the preceding claims, characterized in that the quantity of the compound which can transform nitrocresols into hydrosoluble salts is such that the pH of the aqueous phase during contact is in the range 1 to 9.

4. A process according any one of the preceding claims, characterized in that the compound which can transform nitrocresols into hydrosoluble salts is an alkali metal or alkaline-earth metal hydroxide, carbonate or bicarbonate.

5. A process according to the preceding claim, characterized in that an alkali metal hydroxide is used.

6. A process according to any one of the preceding claims, characterized in that the mixture used originates from the reaction medium from said nitration step which has been separated from the aqueous phase in a decantation-separation step.

7. A process according to any one of claims 1 to 5, characterized in that the reaction mixture from said nitration step is used, which has preferably been separated from the aqueous phase in a decantation-separation step, then washed with water, then separated from the aqueous phase in a decantation-separation step.

8. A process according to any one of the preceding claims, characterized in that the quantity of oxidising agent used is in the range 15 to 1000 moles, more particularly in the range 15 to 500 moles per mole of nitrocresol type compounds present in the mixture.

9. A process according to any one of the preceding claims, characterized in that the organic phase obtained after eliminating the nitrocresols is washed with water and then separated from the organic phase in a decantation-separation step.

10. A process for preparing aromatic amines, characterized in that the process of claim 9 is carried out and the organic phase obtained is dried if necessary then brought into contact with pure or diluted hydrogen in the presence of a hydrogenation catalyst.

## Patentansprüche

1. Verfahren zur Entfernung von Verbindungen vom Nitrocresol-Typ, welche in einem Gemisch auf Basis von Verbindungen vom nitroaromatischen Typ, verschieden von Trinitrotoluolen, enthalten sind, wobei die nitroaromatischen Verbindungen durch Nitrieren von aromatischen Verbindungen mit Salpetersäure in Gegenwart von Schwefelsäure erhalten werden, dadurch gekennzeichnet, daß man das Gemisch eines Teils mit einem Oxidationsmittel, ausgewählt aus Wasserstoffperoxid, Eau de Javel oder deren Mischungen und anderen Teils mit einer Verbindung in Kontakt bringt, welche die Nitrocresole, welche in dem Gemisch enthalten sind, in wasserlösliche Salze umwandeln kann.

2. Verfahren nach dem vorhergehenden Anspruch, dadurch gekennzeichnet, daß man Wasserstoffperoxid als Oxidationsmittel verwendet.

3. Verfahren nach einem der vorhergehenden Ansprüche, dadurch gekennzeichnet, daß die Menge der zur Umwandlung der Nitrocresole in wasserlösliche Salze fähigen Verbindung derart ist, daß der pH-Wert der wäßrigen Phase während des Inkontaktbringens zwischen 1 und 9 enthalten ist.

4. Verfahren nach einem der vorhergehenden Ansprüche, dadurch gekennzeichnet, daß man als Verbindung, die zur Umwandlung der Nitrocresole in wasserlösliche Salze fähig ist, die Alkali-, Erdalkalimetallhydroxide, -carbonate, -bicarbonate verwendet.

5. Verfahren nach dem vorhergehenden Anspruch, dadurch gekennzeichnet, daß man die Alkalimetallhydroxyde verwendet.

6. Verfahren nach einem der vorhergehenden Ansprüche, dadurch gekennzeichnet, daß man ein Gemisch verwendet, daß aus dem Reaktionsmilieu stammt, das aus der vorgenannten Nitrierung herrührt, das vorher von der wäßrigen Phase durch eine Dekantieren-Trennen-Stufe abgetrennt worden ist.

7. Verfahren nach einem der Ansprüche 1 bis 5, dadurch gekennzeichnet, daß man ein aus der vorerwähnten Nitrierung stammendes Reaktionsgemisch verwendet, das vorzugsweise vorher von der wäßrigen Phase durch eine Dekantieren-Trennen-Stufe abgetrennt worden ist, welche mit Wasser gewaschen worden ist, dann von der wäßrigen Phase durch eine Dekantieren-Trennen-Stufe abgetrennt worden ist.

8. Verfahren nach einem der vorhergehenden Ansprüche, dadurch gekennzeichnet, daß man eine Menge an Oxidationsmittel verwendet, welche zwischen 15 und 1.000 Mol enthalten ist, und insbesondere eine Menge, welche zwischen 15 und 500 Mol pro Mol Verbindungen von in dem Gemisch vorliegenden Verbindungen vom Nitrocresoltyp enthalten ist.

9. Verfahren nach einem der vorhergehenden Ansprüche, dadurch gekennzeichnet, daß man die organische Phase, welche man nach dem Entfernen der Nitrocresole erhalten hat, mit Wasser wäscht und diese von der organischen Phase durch eine Dekantieren-Trennen-Stufe abtrennt.

10. Verfahren zur Herstellung von aromatischen Aminverbindungen, dadurch gekennzeichnet, daß man das Verfahren gemäß Anspruch 9 durchführt und man die erhaltene organische Phase, gegebenenfalls vorher getrocknet, in Kontakt mit reinem oder verdünntem Wasserstoff in Gegenwart eines Hydrierkatalisators bringt.
